# EUROPEAN PATENT SPECIFICATION

(11) **EP 3 632 489 B1**
(45) Date of publication and mention of the grant of the patent: **23.08.2023**
(21) Application number: 18306316.3
(22) Date of filing: 05.10.2018
(51) Int. Cl.: A61M 5/32

(54) **CAP REMOVER HAVING GASKET COMPRESSION**
KAPPENENTFERNER MIT DICHTUNGSKOMPRIMIERUNG
EXTRACTEUR DE BOUCHON AYANT LA COMPRESSION DU JOINT D'ÉTANCHÉITÉ

(43) Date of publication of application: 08.04.2020
(73) Proprietor: Becton Dickinson France, 38800 Le Pont-de-Claix (FR)
(72) Inventor: MILLS, Freddy, 38000 GRENOBLE (FR); VALENTIN, Stéphane, 38470 L'ALBENC (FR)
(74) Representative: Germain Maureau

(56) References cited:
- US-A1- 2017 157 334
- US-A1- 2017 165 433
- US-A1- 2017 274 152
- US-A1- 2017 354 789

## Description

### Field of the Invention

The present disclosure relates generally to medical injector devices for delivery of a fluid or liquid medicament. More particularly, the present disclosure relates to a safety cap and/or a cap remover for a medical injector device or a syringe.

### Description of Related Art

Medical injectors and syringes are well known in the art. Medical injectors may include auto-injectors and pen injectors which are capable of delivering selected doses of fluids including liquid medicaments or vaccinations to a patient. Medical injectors typically are configured to receive a standard pre-filled glass or plastic syringe tipped with an injection needle. These devices may include a drive member for advancing a plunger into a syringe barrel to expel a liquid medicament out through the needle. The required manipulation of a standard prior art hypodermic syringe can be inconvenient, particularly where the injection is self-administered in a public environment, and many medication delivery pens, pen injectors, or other self-injectors have been developed to facilitate self-administration of injections. The document US20173 54789 discloses a a cap assembly for a medicament delivery device and the document US2017274152 discloses a needle sheath remover.

In order to maintain sterility prior to use and to reduce the risk of incurring an accidental needle-stick, protection of the needle tip is important. Medical injectors are typically supplied with a rubber or plastic cap which guards the needle prior to use. Immediately prior to use, the user must remove the protective cap from the injector, such as by using the cap protector of the present invention.

### SUMMARY OF THE INVENTION

In accordance with an embodiment of the present invention, a cap remover including an outer member, an activation member at least partially received within the outer member and configured to move relative to the outer member, and an inner member positioned within the outer member and configured to radially compress and to exert a radial force on a cap under an axial pressure force asserted by the activation member that moves the activation member from a first axial position to a second axial position, such that the inner member compresses from a first position to a second position.

In accordance with one embodiment of the present invention, in the first position, the inner member has a first inner diameter and, in the second position, the inner member has a second inner diameter, and wherein the second inner diameter is smaller than the first inner diameter.

The activation member may define at least one retaining recess.

The outer member may include at least one retaining tab.

The activation member may be held in the outer member in a non-activated position when the at least one retaining tab is held in the at least one retaining recess.

The inner member may include a rubber gasket.

At least a portion of the inner member may be positioned between a proximal end of the activation member and an inner projection that extends inwardly from an inner surface of the outer member.

The inner member may include at least one resilient retaining arm that is configured to compress inwardly under the axial pressure force applied by the activation member.

The at least one retaining arm may compress in a radial direction under the axial pressure force asserted by the activation member.

The inner member further may include at least one retaining tab positioned on an end of the at least one retaining arm.

The at least one retaining tab may extend inwardly relative to the at least one retaining arm. The inner member further may include at least one stabilizing arm.

In accordance with one embodiment of the present invention, a medical injector includes an injector housing defining a reservoir, a plunger rod, a stopper engaged with a portion of the plunger rod and slidably disposed within the reservoir, the stopper sized relative to an interior of the injector housing to provide sealing engagement with a sidewall of the injector housing, a needle having a sharpened first end and a second end in communication with the reservoir, a first cap covering the sharpened first end of the needle, and a second cap covering at least a part of the first cap, the second cap including an outer member, an activation member at least partially received within the outer member and configured to move relative to the outer member, and an inner member positioned within the outer member and configured to radially compress and to exert a radial force on a cap under an axial pressure force asserted by the activation member that moves the activation member from a first axial position to a second axial position, such that the inner member compresses from a first position to a second position to exert a radial force on the first cap.

In accordance with one embodiment of the present invention, in the first position, the inner member has a first inner diameter and, in the second position, the inner member has a second inner diameter, and wherein the second inner diameter is smaller than the first inner diameter.

The activation member may define at least one retaining recess.

The outer member may include at least one retaining tab.

The activation member may be held in the outer member in a non-activated position when the at least one retaining tab is held in the at least one retaining recess.

The inner member may include a rubber gasket.

At least a portion of the inner member may be positioned between a proximal end of the activation member and an inner projection that extends inwardly from an inner surface of the outer member.

The inner member may include at least one resilient retaining arm that is configured to compress inwardly under the axial pressure force applied by the activation member.

The at least one retaining arm may compress in a radial direction under the axial pressure force asserted by the activation member.

The inner member further may include at least one retaining tab positioned on an end of the at least one retaining arm.

The at least one retaining tab may extend inwardly relative to the at least one retaining arm.

The inner member further may include at least one stabilizing arm.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIG. 1 is a perspective view of a medical injector with a cap in accordance with an embodiment of the present invention;
FIG. 2 is a perspective view of a medical injector in accordance with an embodiment of the present invention;
FIG. 3 is a cross-sectional view of a medical injector in accordance with an embodiment of the present invention;
FIG. 4 is a cross-sectional view of a cap remover in accordance with an embodiment of the present invention;
FIG. 5 is a cross-sectional view of the cap remover of FIG. 4 illustrating a closing motion for the cap remover;
FIG. 6 is a cross-sectional view of the cap remover of FIG. 4 illustrating a gasket compression of the cap remover;
FIG. 7 is a rear perspective view of a retaining ring of a cap remover in accordance with an embodiment of the present invention;
FIG. 8 is a front perspective view of the retaining ring of FIG. 7;
FIG. 9 is a cross-sectional view of a cap remover including the retaining ring of FIG. 7 in accordance with an embodiment of the present invention; and
FIG. 10 is a cross-sectional view of the cap remover of FIG. 9 in an activated position.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the following discussion, "distal" refers to a direction generally toward an end of a medical injector adapted for contact with a patient and/or engagement with a separate device, and "proximal" refers to the opposite direction of distal, i.e., away from the end of a medical injector adapted for engagement with the separate device. For purposes of this disclosure, the above-mentioned references are used in the description of the components of a medical injector in accordance with the present disclosure.

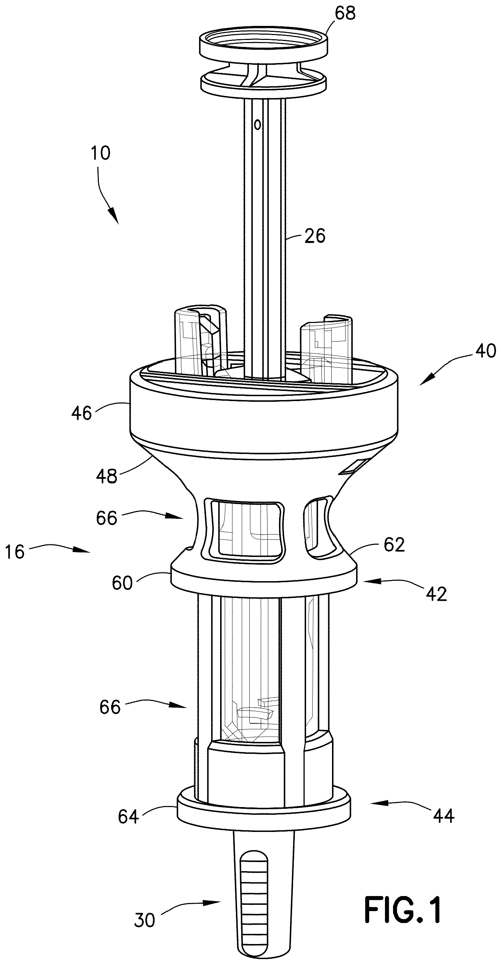

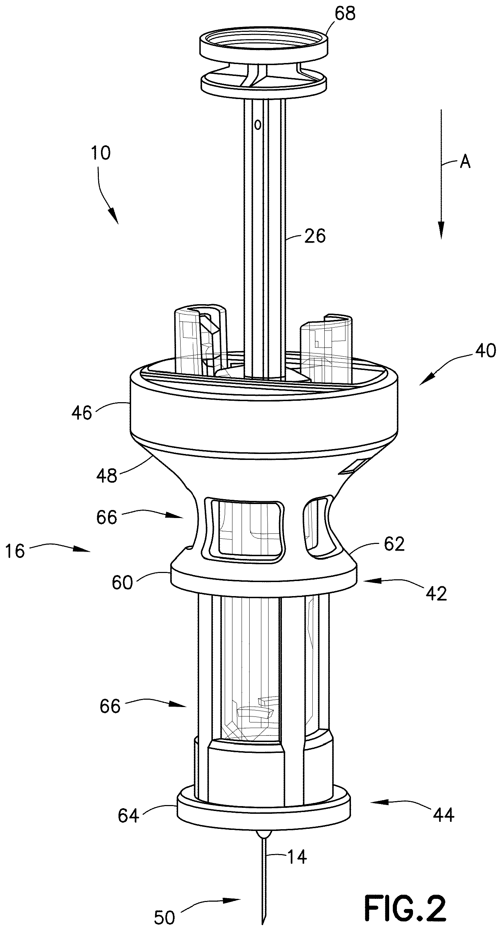

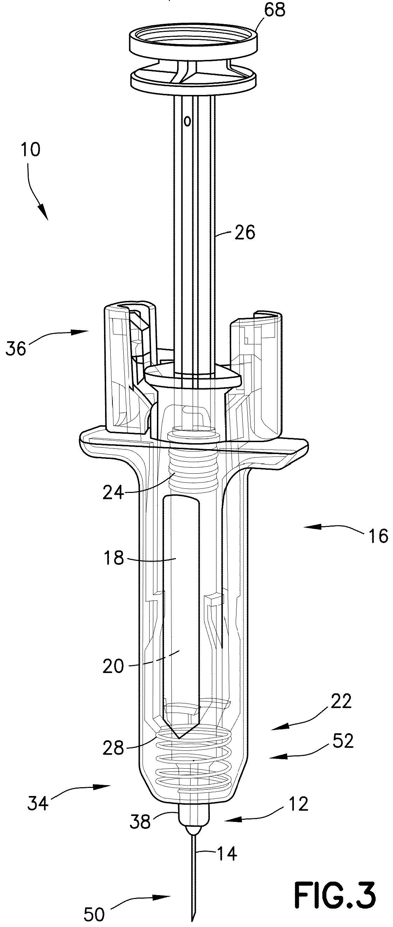

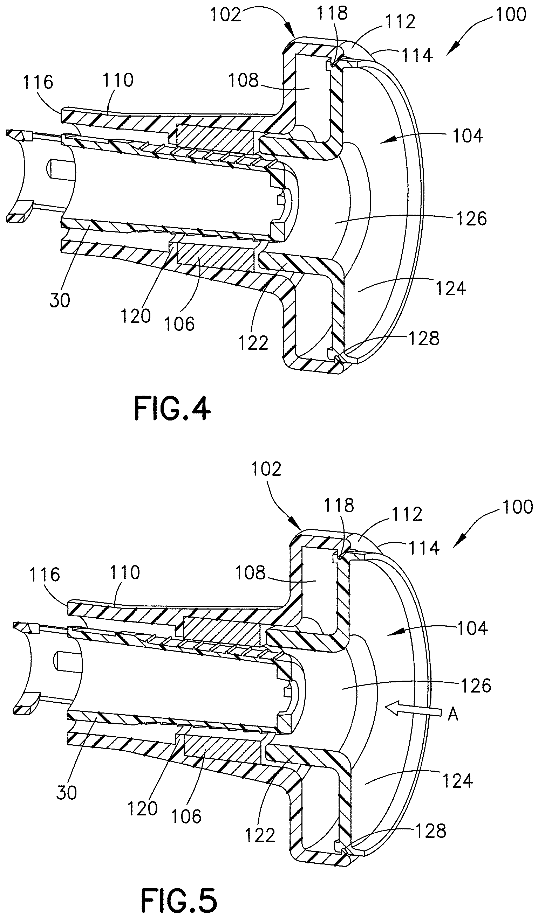

FIGS. 1-3 illustrate an exemplary embodiment of the present disclosure. Referring to FIGS. 1-3, in one embodiment, a medical injector 10 of the present disclosure includes a first cap 30. The first cap 30 may be formed of a plastic, a thermoplastic elastomer, and/or rubber guard material. Referring to FIGS. 2 and 3, the first cap 30 shields and covers a distal end 50 of a needle 14. A second cap or cap remover 100 (shown in FIG. 4) may shield and cover the distal end 50 of the needle 14 and the first cap 30.

In one embodiment, with the first cap 30 covering the distal end 50 of the needle 14 and with the second cap 100 covering the distal end 50 of the needle 14 and the first cap 30, the second cap 100 may be activated such that it engages with the first cap 30 via a compression fit such that removal of the second cap 100 simultaneously removes the first cap 30 from the distal end 50 of the needle 14. More precisely, the second cap 100 surrounds at least partially the first cap 30. The second cap 100 may be positioned in a non-activated position, wherein the second cap 100 surrounds the first cap 30 without compressing the first cap 30 radially, and prefereably without contacting the first cap 30, such that the second cap 100 is free to move independently from the first cap 30, or in an activated position, wherein the second cap 100 compresses radially the first cap 30 such that removal of the second cap 100 simultaneously removes the first cap 30 from the distal end 50 of the needle 14.

Referring to FIGS. 1-3, a medical injector 10 includes a needle assembly 12 having a needle 14, an injector housing 16, a barrel 18 having a reservoir 20 for medicament sealed by a septum 22, a stopper 24, a plunger rod 26 having a flange 68, a spring 28, a first cap 30, and a second cap or a cap remover 100.

The medical injector 10 includes a distal end 34 and a proximal end 36. The reservoir 20 of the barrel 18 is encased within the injector housing 16. In one embodiment, the reservoir 20 may be defined by the injector housing 16. In another embodiment, the reservoir 20 may be defined by a separate component contained within the injector housing 16, e.g., a cartridge or barrel. The needle assembly 12 includes a needle 14 and a hub 38. The needle 14 includes a distal end 50, formed for insertion into a patient, and a proximal end 52.

The medical injector 10 of the present disclosure may be of various forms, including being a syringe, self-injector, auto-injector, manual injector, or pen injector. In one embodiment, the medical injector 10 is well-suited for administering at least one fixed dose. In another embodiment, the medical injector 10 is well-suited for administering a series of fixed doses. The medical injector 10 may be configured in any way known to be compatible with the plunger rod 26. The medical injector 10 may include a reservoir 20 for accommodating an injectable medicament, which may be a drug cartridge or formed directly in the medical injector 10. The reservoir 20 may have one or more stoppers 24 associated therewith.

Referring to FIGS. 1-3, the injector housing 16 of the medical injector 10 includes an upper guard 40, a lower guard 42, and a base 44. The upper guard 40 includes a first flange 46 and a first tapered wall 48. In one embodiment, the upper guard 40 provides a gripping component that includes surfaces for accommodating a user's fingers, such as finger grip indentations or similar structure. For example, the upper guard 40 provides ergonomically shaped surfaces that substantially conform to a user's fingertips to aid the user in manipulating the medical injector 10 and using the medical injector 10 in a medical procedure, and may provide multiple finger grip positions for the user. The upper guard 40 allows a user to handle and/or grip the medical injector 10 in a variety of different ways.

The lower guard 42 includes a second flange 60 and a second tapered wall 62. The lower guard 42 provides a second gripping component that includes surfaces for accommodating a user's fingers, such as finger grip indentations or similar structure. In one embodiment, the lower guard 42 provides a user with vertical stability in hand, precisely during needle insertion with the medical injector 10.

The upper guard 40 and the lower guard 42 together provide a gripping component that includes surfaces for accommodating a user's fingers to aid the user in manipulating the medical injector 10 and using the medical injector 10 in a medical procedure.

The base 44 includes a flange 64 that contacts the skin of a user during use of the medical injector 10. The base 44 provides a stability component that stabilizes the medical injector 10 on a user's skin during use of the medical injector 10. In one embodiment, the base 44 also hides the spring 28.

Referring to FIGS. 1-3, the injector housing 16, the upper guard 40, the lower guard 42, and the base 44 are configured to provide viewing windows 66. The viewing windows 66 allow a user to see the barrel 18 that includes the medicament.

Referring to FIG. 1, a first cap 30 shields and covers the distal end 50 of the needle 14. The second cap or cap remover 100 shields and covers the distal end 50 of the needle 14 and the first cap 30. In one embodiment, the second cap 100 may be the only cap that shields and covers the distal end 50 of the needle 14.

With reference to FIG. 4, the second cap or cap remover 100 in accordance with an embodiment of the present invention is described in greater detail. The second cap or cap remover 100 is configured to assist a user in removing the first cap 30 from a medical injector 10. The second cap or cap remover 100 includes an outer member 102, an activation member 104, and an inner member 106. The outer member 102 and the activation member 104 may be made of a medical grade plastic. The inner member 106 may be made of rubber, thermoplastic elastomer, a flexible plastic, or any other material capable of compressing under pressure. In one embodiment, the activation member 104 and the inner member 106 are received within a cavity 108 defined by the outer member 102.

The outer member 102 may include a body 110 and a base 112 formed as a monolithic component. In one embodiment, the base 112 has a greater diameter than the body 110. The cavity 108 defined by the outer member 102 extends from a distal end 114 of the outer member 102 to a proximal end 116 of the outer member 102. The cavity 108 is configured to receive at least a portion of the first cap 30. At least a portion of an inner surface of the base 112 includes an inwardly-projecting retaining tab 118, preferably located at a distal end of the outer member 102 and, more preferably, at a distal end of the base 112 of the outer member 102. In one embodiment, the retaining tab 118 extends around the entire inner circumference of the base 112. In one embodiment, the retaining tab 118 extends around a portion of the inner circumference of the base 112. In one embodiment, a plurality of retaining tabs 118 are spaced along the inner circumference of the base 112. The outer member 102 also includes an inner projection 120 that extends inwardly from an inner surface of the body 110. The inner projection 120 creates a reduction in the diameter of a portion of the cavity 108 of the body 110 of the outer member 102. Therefore, the portion of the cavity 108 defined in the body 110 has a smaller diameter towards a proximal end of the body 110 and a wider diameter towards a distal end of the body 110.

With reference to FIGS. 4-6, the activation member 104 of the second cap or cap remover 100 in accordance with an embodiment of the present invention is described in greater detail. The activation member 104 includes a body 122 and a base 124. In one embodiment, the general shape of the activation member 104 corresponds to the general shape of the outer member 102. The activation member 104 is generally received within the outer member 102. The body 122 of the activation member 104 is received within the body 110 of the outer member 102. In one embodiment, a diameter of the body 122 is less than a diameter of the base 124. The activation member 104 also defines a cavity 126 that extends from a proximal end of the activation member 104 to a distal end of the activation member 104. In one embodiment, the cavity 126 is configured to receive at least a portion of the first cap 30. A retaining recess 128 is defined in an outer surface of the base 124. The retaining recess 128 is configured to receive the retaining tab 118 projecting from the base 112 of the outer member 102. The retaining tab 118 may be received in a snap-fit arrangement with the retaining recess 128. In one embodiment, the retaining recess 128 extends around the entire outer circumference of the base 124. In one embodiment, the retaining recess 128 extends around a portion of the outer circumference of the base 124. In one embodiment, a plurality of recesses 128 are defined in the outer circumference of the base 124. The activation member 104 may be positioned in a non-activated position within the outer member 102 when the retaining tab 118 is held in the retaining recess 128. The activation member 104 may also be positioned in an activated position with respect to the outer member 102 when the retaining tab 118 is located distally from the base 124 of the activation member 104, such that the base 124 of the activation member 104 abuts against the retaining tab 118.

With reference to FIGS. 4-6, the inner member 106 in accordance with an embodiment of the present invention is described in greater detail. The inner member 106 is positioned within the body 110 of the outer member 102. The inner member 106 may be held between a proximal end of the body 122 of the activation member 104 and the inner projection 120 of the body 110 of the outer member 102. In one embodiment, the inner member 106 may be a rubber gasket that is configured to compress under pressure. The inner member 106 may be a rubber gasket ring. In one embodiment, the inner member 106 may be a plurality of inner members that are distributed around the inner circumference of the body 110. It is contemplated that the inner member 106 may be any member that is capable of at least compressing in a radial direction under an axial pressure such that an inner diameter of the inner member is reduced. In one embodiment, the inner member 106 may be configured to compress under at least an axial pressure. As the axial pressure is applied to the inner member 106, the inner member 106 is axially compressed such that an inner diameter of the inner member 106 is reduced.

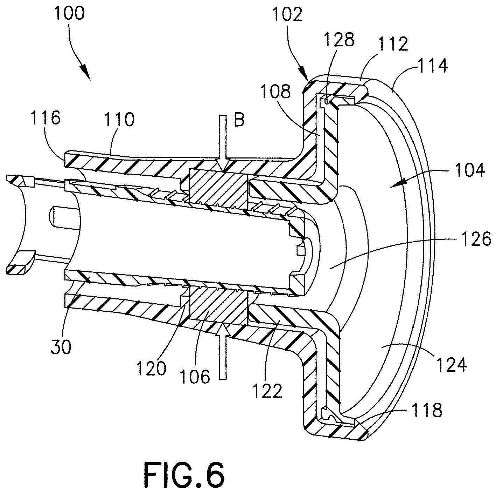

With reference to FIGS. 5 and 6, use of the second cap or cap remover 100 in accordance with an embodiment of the present invention is described in greater detail. In a first step, the second cap or cap remover 100 is positioned on the first cap 30 of the medical injector 10. The first cap 30 is inserted into the proximal end of the outer member 102 so the first cap 30 extends into the cavity 108 defined in the outer member 102. The second cap or cap remover 100 continues to be pushed onto the first cap 30 from a first position to a second position such that a portion of the first cap 30 eventually extends through the inner member 106 and into the cavity 126 defined by the activation member 104. The inner member 106 does not radially compress the first cap 30 as long as the activation member 104 is in a non-activated position. Consequently, the second cap 100 may move independently from the first cap 30 as long as the activation member 104 is in a non-activated position.

To activate the activation member 104, an axial pressure force A may be applied against the base 124 of the activation member 104. The axial pressure force A causes the activation member 104 to move in a proximal direction towards the outer member 102 from a first position to a second position. In the first position, the retaining tab 118 is preferably inserted into the retaining recess 128. Once a sufficient force has been applied to the base 124 of the activation member 104, the fitted arrangement between the retaining tab 118 of the outer member 102 and the retaining recess 128 of the activation member 104 is broken such that the activation member 102 moves relative to the outer member 102 within the cavity 108 defined by the outer member 102. In the second position, the retaining tab 118 is preferably positioned distally from the base 124 of the activation member 104.

As the activation member 104 is pushed in an axial direction, the proximal end of the body 122 of the activation member 104 abuts the inner member 106 held in the cavity 108 defined by the outer member 102. The axial pressure asserted against the inner member 106 causes the inner member 106 to compress between the proximal end of the activation member 104 and the inner projection 120 of the outer member 102. Axial compression of the inner member 106 causes the inner diameter of the inner member 106 to decrease in a radial direction. The compression of the inner member 106 in the radial direction creates a radial force B that is applied to an outer surface of the first cap 30. The inner diameter of the inner member 106 radially compresses the first cap 30 therein to create a friction fit between the inner member 106 and the first cap 30. Once the inner member 106 has compressed on to the first cap 30, the user can pull the outer member 102 in an axial distal direction such that the first cap 30 is pulled along with the second cap or cap remover 100 due to the friction fit between the inner member 106 and the first cap 30.

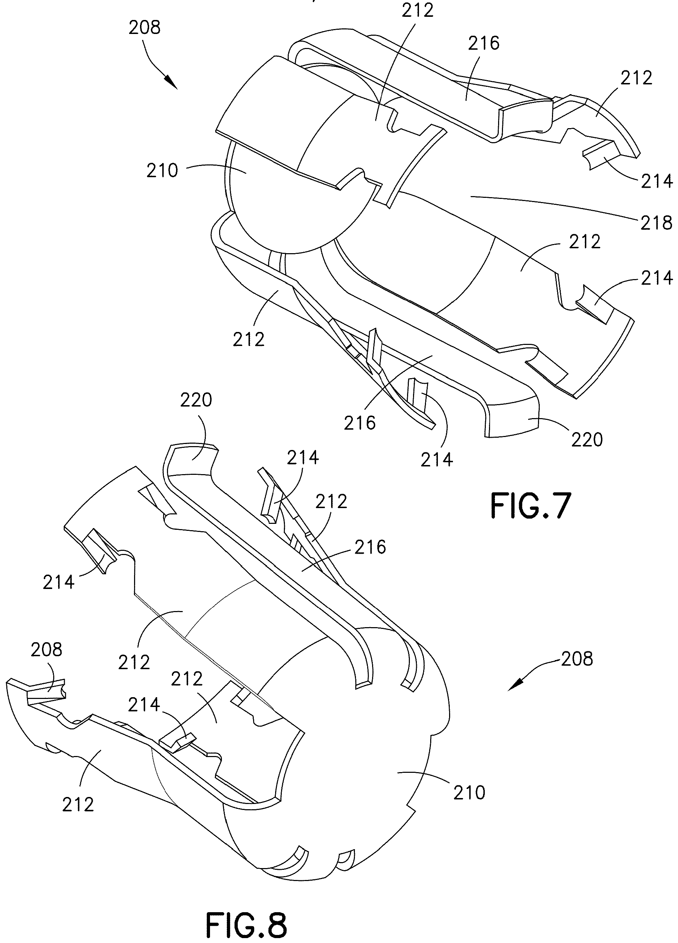

With reference to FIGS. 7-10, a second cap or cap remover 200 according to another embodiment of the present invention is described in greater detail. The second cap or cap remover 200 includes a similar construction to the second cap or cap remover 100 described above, but includes several structural differences. The second cap or cap remover 200 includes an outer member 202 and an activation member 204 at least partially received within a cavity 206 defined by the outer member 202. An inner member 208 is positioned within the cavity 206 defined by the outer member 202. The inner member 208 is provided to assist in removing the first cap 30 from the medical injector 10. When the activation member 204 is activate, the inner member 208 is configured to receive a distal end of the first cap 30 therein, such that, as the second cap or cap remover 200 is moved in an axial distal direction, the first cap 30 moves with the inner member 208 to remove the first cap 30 from the medical injector 10, as will be described in greater detail below. When the activation member 204 is not activated, the activation member 204 does not contact and/or compress the first cap 30, such that the cap remover 200 may move without moving the first cap 30 so that the integrity of the sterile barrier formed by the first cap 30 is not damaged.

With reference to FIGS. 7 and 8, the inner member 208 in accordance with an embodiment of the present invention is described in greater detail. The inner member 208 includes a cap 210, a plurality of retaining arms 212 that extend from the cap 210, and a plurality of retaining tabs 214 that extend from ends of the retaining arms 212. In one embodiment, at least one stabilizing arm 216 also extends from the cap 210. The cap 210, retaining arms 212, retaining tabs 214, and stabilizing arms 216 may be formed as a monolithic structure. They may be made of metal. The inner member 208 of the second cap or cap remover 200 is preferably made of metal. The outer member 202 and the activation member 204 of the second cap or cap remover 200 are preferably made of plastic. The second cap or cap remover 200 defines a cavity 218 configured to receive the first cap 30 of the medical injector 10.

Each retaining arm 212 has a first end that is formed with the cap 210 and an opposing end that includes the retaining tabs 214. In one embodiment, the second cap or cap remover 200 includes at least four retaining arms 212. In one embodiment, two retaining arms 212 are circumferential separated from two additional retaining arms 212 by two stabilizing arms 216. It is contemplated that the retaining arms 212 may be circumferentially spaced around the cap 210 from one another at any desired angle. In one embodiment, the retaining arms 212 are spaced equidistantly around the circumference of the cap 210. In one embodiment, at least a portion of each retaining arm 212 extends resiliently outward in a radial direction from the cap 210. Under a certain amount of force, the retaining arms 212 can be pushed inwardly in a radial direction and will return to their initial resting position once the force has been released against the retaining arms 212. The retaining tabs 214 are angled radially inward from a proximal end of the retaining arms 212. The retaining tabs 214 are substantially rigid and do not deform under pressure. The stabilizing arms 216 are confirmed to assist in stabilizing the second cap or cap remover 200 against the first cap 30 when the first cap 30 is inserted into the second cap or cap remover 200. Each stabilizing arm 216 may also include a lip 220 that may abut an inner projection 222 on the outer member 202 of the second cap or cap remover 200 to prevent the inner member 208 from move too far in a proximal direction within the second cap or cap remover 200.

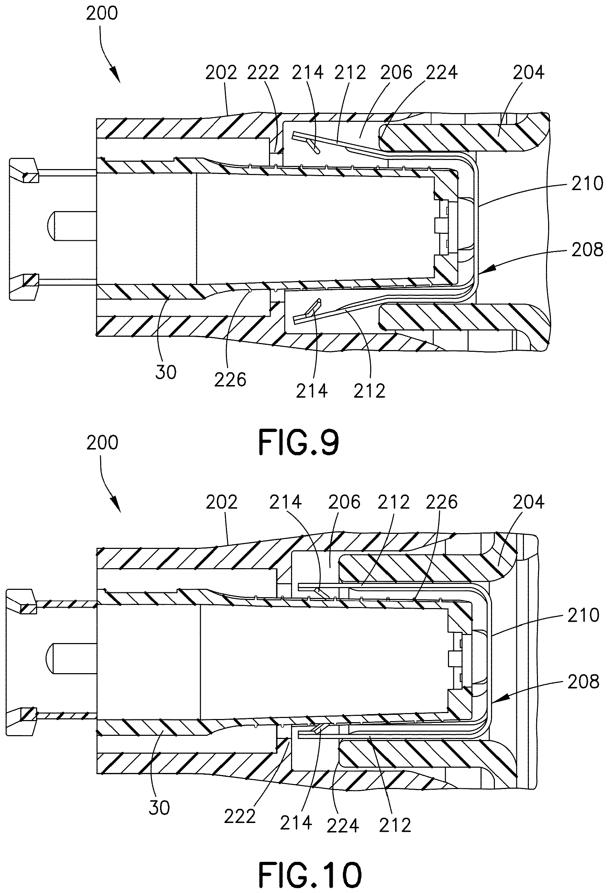

With reference to FIGS. 9 and 10, use of the second cap or cap remover 200 in accordance with one embodiment of the present invention is described in greater detail. The second cap or cap remover 200 is moved in a proximal axial direction relative to the medical injector 10 to position the second cap or cap remover 200 on the first cap 30. As the second cap or cap remover 200 is moved in a proximal direction, the first cap 30 is received in the cavity 206 defined by the outer member 202 and eventually the inner member 208. In one embodiment, the first cap 30 may be inserted into the inner member 208 until the distal end of the first cap 30 abuts the cap 210 of the inner member 208.

As long as the second cap 200 is not activated, the inner member 208 does not compress radially the first cap 30. According to one embodiment, as long as the second cap 200 is not activated, the second cap 200 does not contact the first cap 30.

To activate the second cap or cap remover 200, a proximal axial force is applied against the activation member 204 of the second cap or cap remover 200. The force against the activation member 204 causes the activation member 204 to move in a proximal direction into the cavity 206 relative to the outer member 202. As the activation member 204 is pushed in the proximal direction, a proximal end 224 of the activation member 204 begins to contact the retaining arms 212 of the inner member 208. Since the retaining arms 212 extend radially outward relative to the cap 210 of the inner member 208, the retaining arms 212 extend into the cavity 206 between the proximal end 224 of the activation member 204 and the inner projection 222 of the outer member 202. As the activation member 204 is pushed farther in the proximal direction, the proximal end 224 of the inner member 208 pushes the retaining arms 212 inwardly in a radial direction towards the first cap 30.

As the retaining arms 212 are moved towards the first cap 30, the retaining tabs 214 come into contact with the outer surface of the first cap 30. Once the retaining arms 212 have been completely compressed by the activation member 204, the retaining tabs 214 are pressed against the first cap 30 in a position to pull the first cap 30 from the medical injector 10. In one embodiment, the retaining tabs 214 are configured to abut and engage protrusions or threads 226 extending from an outer surface of the first cap 30. The second cap or cap remover 200 is not activated. Therefore, as the second cap or cap remover 200 is pulled in a distal axial direction relative to the medical injector 10, the retaining tabs 214 abut the protrusions or threads 226 on the first cap 30 to move the first cap 30 along with the second cap or cap remover 200. Therefore, the first cap 30 is moved in a distal axial direction with the second cap or cap remover 200 to remove the first cap 30 from the medical injector 10. It is to be understood that any structures may be used for the retaining tabs 214 and the protrusion or threads 226 on the first cap 30 so long as the retaining tabs 214 are configured to engage the protrusion or threads 226 on the first cap 30 to move the first cap 30 in the distal axial direction along with the second cap or cap remover 200. It is also contemplated that the retaining tabs 214 are configured to be strong enough the clamp the first cap 30 therebetween in a friction fit to pull the first cap 30 from the medical injector 10.

All of the components of the medical injector 10 may be constructed of any known material, and are desirably constructed of medical-grade polymers.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A cap remover, comprising:
an outer member (102, 202);
an inner member (106; 208),
and an activation member (104, 204) at least partially received within the outer member **characterized in that** the activation member is configured to move relative to the outer member; and
the inner member (106, 208) is positioned within the outer member and configured to radially compress and to exert a radial force on a cap (30) under an axial pressure force asserted by the activation member that moves the activation member from a first axial position to a second axial position, such that the inner member compresses from a first position to a second position.

2. The cap remover of claim 1,
wherein, in the first position, the inner member (106, 208) has a first inner diameter and, in the second position, the inner member has a second inner diameter, and
wherein the second inner diameter is smaller than the first inner diameter.

3. The cap remover according to any of the previous claims,
wherein the activation member (104, 204) defines at least one retaining recess (128),
wherein the outer member (102, 202) comprises at least one retaining tab (118), and
wherein the activation member (104, 204) is held in the outer member (102, 202) in a non-activated position when the at least one retaining tab (118) is held in the at least one retaining recess (128).

4. The cap remover of any of the previous claims, wherein the inner member (106) comprises a rubber gasket.

5. The cap remover of claim 1, wherein the inner member (208) comprises at least one resilient retaining arm (212) that is configured to compress inwardly under the axial pressure force applied by the activation member (204).

6. The cap remover of claim 5, wherein the at least one retaining arm (212) compresses in a radial direction under the axial pressure force asserted by the activation member (204).

7. The cap remover of any of claims 5 or 6, wherein the inner member (208) further comprises at least one retaining tab (214) positioned on an end of the at least one retaining arm (212).

8. The cap remover of any of claims 5 to 7, wherein the at least one retaining tab (214) extends inwardly relative to the at least one retaining arm (212).

9. The cap remover of any of claims 6-8, wherein the inner member (208) further comprises at least one stabilizing arm (216).

10. The cap remover of any of the previous claims, wherein at least a portion of the inner member is positioned between a proximal end (122, 124) of the activation member (104, 204) and an inner projection (120, 222) that extends inwardly from an inner surface of the outer member (102, 202).

11. A medical injector, comprising:
an injector housing (16) defining a reservoir (20);
a plunger rod (26);
a stopper (24) engaged with a portion of the plunger rod (26) and slidably disposed within the reservoir (20), the stopper (24) sized relative to an interior of the injector housing (16) to provide sealing engagement with a sidewall of the injector housing (16);
a needle (14) having a sharpened first end and a second end in communication with the reservoir (20);
the cap (30) covering the sharpened first end of the needle (14); and
the cap remover of any of the previous claims covering at least a part of the cap (30).

12. The medical injector of claim 11,
wherein, in the first position, the inner member (106, 208) has a first inner diameter and, in the second position, the inner member (106, 208) has a second inner diameter, and
wherein the second inner diameter is smaller than the first inner diameter.

13. The medical injector of any of claims 11 or 12,
wherein the activation member (104, 204) defines at least one retaining recess (128),
wherein the outer member (102, 202) comprises at least one retaining tab (118), and
wherein the activation member (104, 204) is held in the outer member (102, 202) in a non-activated position when the at least one retaining tab (118) is held in the at least one retaining recess (128).

14. The medical injector of any of claims 11 to 13, wherein the inner member (106) comprises a rubber gasket.

15. The medical injector of claim 11, wherein the inner member (208) comprises at least one resilient retaining arm (212) that is configured to compress under the axial pressure force applied by the activation member (204).

## Patentansprüche

1. Kappenentferner, der Folgendes umfasst:
ein äußeres Element (102, 202);
ein inneres Element (106, 208); und
ein Aktivierungselement (104, 204), das mindestens teilweise im äußeren Element aufgenommen ist, **dadurch gekennzeichnet, dass** das Aktivierungselement so konfiguriert ist, dass es sich im Verhältnis zum äußeren Element bewegt; und das innere Element (106, 208) innerhalb des äußeren Elements positioniert und so konfiguriert ist, dass es radial komprimiert wird und eine radiale Kraft auf eine Kappe (30) unter einer axialen Druckkraft ausübt, die von dem Aktivierungselement ausgeübt wird und die das Aktivierungselement von einer ersten axialen Position in eine zweite axiale Position bewegt, so dass das innere Element von einer ersten Position in eine zweite Position komprimiert wird.

2. Kappenentferner nach Anspruch 1,
wobei das innere Element (106, 208) in der ersten Position einen ersten Innendurchmesser aufweist und das innere Element in der zweiten Position einen zweiten Innendurchmesser aufweist, und
wobei der zweite Innendurchmesser kleiner ist als der erste Innendurchmesser.

3. Kappenentferner nach einem der vorhergehenden Ansprüche,
wobei das Aktivierungselement (104, 204) mindestens eine Halteaussparung (128) definiert,
wobei das äußere Element (102, 202) mindestens eine Haltelasche (118) umfasst, und
wobei das Aktivierungselement (104, 204) im äußeren Element (102, 202) in einer nicht aktivierten Position gehalten wird, wenn die mindestens eine Haltelasche (118) in der mindestens einen Halteaussparung (128) gehalten wird.

4. Kappenentferner nach einem der vorhergehenden Ansprüche, wobei das innere Element (106) eine Gummidichtung umfasst.

5. Kappenentferner nach Anspruch 1, wobei das innere Element (208) mindestens einen elastischen Haltearm (212) umfasst, der so konfiguriert ist, dass er unter der von dem Aktivierungselement (204) ausgeübten axialen Druckkraft nach innen komprimiert wird.

6. Kappenentferner nach Anspruch 5, wobei der mindestens eine Haltearm (212) unter der von dem Aktivierungselement (204) ausgeübten axialen Druckkraft in radialer Richtung komprimiert wird.

7. Kappenentferner nach einem der Ansprüche 5 oder 6, wobei das innere Element (208) ferner mindestens eine Haltelasche (214) umfasst, die an einem Ende des mindestens einen Haltearms (212) positioniert ist.

8. Kappenentferner nach einem der Ansprüche 5 bis 7, wobei sich die mindestens eine Haltelasche (214) im Verhältnis zu dem mindestens einen Haltearm (212) nach innen erstreckt.

9. Kappenentferner nach einem der Ansprüche 6-8, wobei das innere Element (208) ferner mindestens einen Stabilisierungsarm (216) umfasst.

10. Kappenentferner nach einem der vorhergehenden Ansprüche, wobei mindestens ein Abschnitt des inneren Elements zwischen einem proximalen Ende (122, 124) des Aktivierungselements (104, 204) und einem inneren Vorsprung (120, 222) positioniert ist, der sich von einer Innenfläche des äußeren Elements (102, 202) nach innen erstreckt.

11. Medizinischer Injektor, der Folgendes umfasst:
ein Injektorgehäuse (16), das ein Reservoir (20) definiert;
eine Kolbenstange (26);
einen Stopfen (24), der mit einem Abschnitt der Kolbenstange (26) in Eingriff steht und verschiebbar innerhalb des Reservoirs (20) angeordnet ist, wobei der Stopfen (24) im Verhältnis zum Inneren des Injektorgehäuses (16) so dimensioniert ist, dass er einen abdichtenden Eingriff mit einer Seitenwand des Injektorgehäuses (16) bereitstellt;
eine Nadel (14) mit einem geschärften ersten Ende und einem zweiten Ende in Verbindung mit dem Reservoir (20);
wobei die Kappe (30) das geschärfte erste Ende der Nadel (14) abdeckt; und
der Kappenentferner nach einem der vorherigen Ansprüche, der mindestens einen Abschnitt der Kappe (30) abdeckt.

12. Medizinischer Injektor nach Anspruch 11,
wobei das innere Element (106, 208) in der ersten Position einen ersten Innendurchmesser aufweist und das innere Element (106, 208) in der zweiten Position einen zweiten Innendurchmesser aufweist, und
wobei der zweite Innendurchmesser kleiner ist als der erste Innendurchmesser.

13. Medizinischer Injektor nach einem der Ansprüche 11 oder 12,
wobei das Aktivierungselement (104, 204) mindestens eine Halteaussparung (128) definiert,
wobei das äußere Element (102, 202) mindestens eine Haltelasche (118) umfasst, und
wobei das Aktivierungselement (104, 204) im äußeren Element (102, 202) in einer nicht aktivierten Position gehalten wird, wenn die mindestens eine Haltelasche (118) in der mindestens einen Halteaussparung (128) gehalten wird.

14. Medizinischer Injektor nach einem der Ansprüche 11 bis 13, wobei das innere Element (106) eine Gummidichtung umfasst.

15. Medizinischer Injektor nach Anspruch 11, wobei das innere Element (208) mindestens einen elastischen Haltearm (212) umfasst, der so konfiguriert ist, dass er unter der von dem Aktivierungselement (204) ausgeübten axialen Druckkraft komprimiert wird.

## Revendications

1. Dispositif d'enlèvement de capuchon, comprenant :
un élément extérieur (102, 202) ;
un élément intérieur (106 ; 208), et
un élément d'activation (104, 204) reçu au moins partiellement à l'intérieur de l'élément extérieur, **caractérisé en ce que** l'élément d'activation est configuré pour se déplacer par rapport à l'élément extérieur ; et
l'élément intérieur (106, 208) est positionné à l'intérieur de l'élément extérieur et configuré pour se comprimer radialement et pour exercer une force radiale sur un capuchon (30) sous une force de pression axiale exercée par l'élément d'activation qui déplace l'élément d'activation d'une première position axiale vers une deuxième position axiale, de sorte que l'élément intérieur se comprime d'une première position vers une deuxième position.

2. Dispositif d'enlèvement de capuchon de la revendication 1,
dans lequel, dans la première position, l'élément intérieur (106, 208) a un premier diamètre intérieur et, dans la deuxième position, l'élément intérieur a un deuxième diamètre intérieur, et
dans lequel le deuxième diamètre intérieur est inférieur au premier diamètre intérieur.

3. Dispositif d'enlèvement de capuchon de l'une des revendications précédentes,
dans lequel l'élément d'activation (104, 204) définit au moins un évidement de retenue (128),
dans lequel l'élément extérieur (102, 202) comprend au moins une languette de retenue (118), et
dans lequel l'élément d'activation (104, 204) est maintenu dans l'élément extérieur (102, 202) dans une position non activée lorsque l'au moins une languette de retenue (118) est maintenue dans l'au moins un évidement de retenue (128).

4. Dispositif d'enlèvement de capuchon de l'une des revendications précédentes, dans lequel l'élément intérieur (106) comprend un joint en caoutchouc.

5. Dispositif d'enlèvement de capuchon de la revendication 1, dans lequel l'élément intérieur (208) comprend au moins un bras de retenue élastique (212) qui est configuré pour se comprimer vers l'intérieur sous la force de pression axiale appliquée par l'élément d'activation (204).

6. Dispositif d'enlèvement de capuchon de la revendication 5, dans lequel l'au moins un bras de retenue (212) se comprime dans une direction radiale sous la force de pression axiale exercée par l'élément d'activation (204).

7. Dispositif d'enlèvement de capuchon de l'une des revendications 5 ou 6, dans lequel l'élément intérieur (208) comprend en outre au moins une languette de retenue (214) positionnée sur une extrémité de l'au moins un bras de retenue (212).

8. Dispositif d'enlèvement de capuchon de l'une des revendications 5 à 7, dans lequel l'au moins une languette de retenue (214) s'étend vers l'intérieur par rapport à l'au moins un bras de retenue (212).

9. Dispositif d'enlèvement de capuchon de l'une des revendications 6 à 8, dans lequel l'élément intérieur (208) comprend en outre au moins un bras de stabilisation (216).

10. Dispositif d'enlèvement de capuchon de l'une des revendications précédentes, dans lequel au moins une partie de l'élément intérieur est positionnée entre une extrémité proximale (122, 124) de l'élément d'activation (104, 204) et une saillie intérieure (120, 222) qui s'étend vers l'intérieur d'une surface intérieure de l'élément extérieur (102, 202).

11. Injecteur médical, comprenant :
un boîtier d'injecteur (16) définissant un réservoir (20) ;
une tige de piston (26) ;
un piston (24) en prise avec une partie de la tige de piston (26) et disposée de manière coulissante à l'intérieur du réservoir (20), la butée (24) étant dimensionnée par rapport à un intérieur du boîtier d'injecteur (16) pour assurer une prise étanche avec une paroi latérale du boîtier d'injecteur (16) ;
une aiguille (14) ayant une première extrémité pointue et une deuxième extrémité en communication avec le réservoir (20) ;
le capuchon (30) recouvrant la première extrémité pointue de l'aiguille (14) ; et
le dispositif d'enlèvement de capuchon de l'une des revendications précédentes recouvrant au moins une partie du capuchon (30).

12. Injecteur médical de la revendication 11,
dans lequel, dans la première position, l'élément intérieur (106, 208) a un premier diamètre intérieur et, dans la deuxième position, l'élément intérieur (106, 208) a un deuxième diamètre intérieur, et
dans lequel le deuxième diamètre intérieur est inférieur au premier diamètre intérieur.

13. Injecteur médical de l'une des revendications 11 ou 12,
dans lequel l'élément d'activation (104, 204) définit au moins un évidement de retenue (128),
dans lequel l'élément extérieur (102, 202) comprend au moins une languette de retenue (118), et
dans lequel l'élément d'activation (104, 204) est maintenu dans l'élément extérieur (102, 202) dans une position non activée lorsque l'au moins une languette de retenue (118) est maintenue dans l'au moins un évidement de retenue (128).

14. Injecteur médical de l'une des revendications 11 à 13, dans lequel l'élément intérieur (106) comprend un joint en caoutchouc.

15. Injecteur médical de la revendication 11, dans lequel l'élément intérieur (208) comprend au moins un bras de retenue élastique (212) qui est configuré pour se comprimer sous la force de pression axiale appliquée par l'élément d'activation (204).
